# EUROPEAN PATENT APPLICATION

(11) **EP 4 765 865 A1**
(43) Date of publication of application: **24.06.2026**
(21) Application number: 25224727.5
(22) Date of filing: 17.12.2025
(51) Int. Cl.: H04R 1/10, H04R 25/00

(54) **COMMUNICATION DEVICE FOR A HEARING PROTECTION APPARATUS WITH MODULAR WIRELESS FUNCTIONALITY CONTROL AND METHOD THERETO**

(30) Priority: 19.12.2024 EP 24221433
(71) Applicant: FalCom A/S, 2750 Ballerup (DK)
(72) Inventor: ARGUE, Colin, 2750 Ballerup (DK); KIRKELUND, Jens, 2750 Ballerup (DK)
(74) Representative: Aera A/S

(57) **Abstract**

A communication device for a hearing protection apparatus comprising a hearing protection device is disclosed. The communication device comprises a processing unit, a memory, a radio interface, and a hearing protection device interface. The communication device also comprises a modular button assembly comprising a wireless interface. The processing unit of the communication device is configured to obtain a first input from the hearing protection device and/or transmit a first output to the hearing protection device via the hearing protection device interface. The radio interface comprises a radio connector that comprises terminals for wired connection to a radio unit. The processing unit is configured to obtain a second input from the radio unit and/or transmit a second output to the radio unit via the radio interface.

## Description

The present disclosure relates to a communication device, and a method for producing the same, for a hearing protection device that provides for with modular wireless functionality control.

### BACKGROUND

Hearing protection systems or devices may be configured with wireless control functionality, such as the ability to control a user headset with a wireless or remote controller. Other similar systems may be controlled with a wired controller. Depending on the needs or preferences of a user, a hearing protection system may thus be controlled wirelessly or with a wired control unit. Support for producing two units that are similar but for their wireless control functionality creates manufacturing and supply challenges.

### SUMMARY

Certain hearing protection products, for example, from a common product line or with otherwise similar features may include wireless control functionality while other such products do not. To satisfy both interests for wireless and wired control, product manufacturing may be duplicated to provide multiple lines of products. This may lead to bloated processes and supply issues, create inventory inefficiencies, and create duplicative design efforts.

Designing a singular solution that is one that supports both wired and wireless functionality may, however, introduce undesirable performance that negatively impacts hearing protection in certain circumstances. For example, a wireless controller or button assembly may use a wireless signal that interferes with other aspects of a controller. Such issues may not be present in wired controllers.

Accordingly, there is a need for communication devices and methods for manufacturing the same that mitigate, alleviate, or address the shortcomings existing and provide for modular wireless functionality control. Devices and methods providing such solutions are disclosed herein.

A communication device for a hearing protection apparatus comprising a hearing protection device is disclosed. The communication device comprises a processing unit, a memory, a radio interface, and a hearing protection device interface. The processing unit is configured to obtain a first input from the hearing protection device and/or transmit a first output to the hearing protection device via the hearing protection device interface. The radio interface optionally comprises a radio connector. The radio connector comprises terminals for wired connection to a radio unit. The processing unit is configured to obtain a second input from the radio unit and/or transmit a second output to the radio unit via the radio interface. The radio interface may be a digital radio interface. The communication device may comprise a modular button assembly comprising a wireless interface.

A method for manufacturing a communication device of a hearing protection apparatus comprising a hearing protection device is disclosed. The communication device comprises a processing unit, a memory, a radio interface comprising a radio connector. The radio connector comprises terminals for wired connection to a radio unit and a hearing protection device interface. The communication device comprises a modular button assembly comprising a wireless interface. The method comprises mounting and/or sealing the modular button assembly to a housing of the communication device or to an aspect or component of the communication device.

**It** is an advantage of the present disclosure that a communication device may accept, and may be fitted with, a modular button assembly to allow for wireless or wired control options in a common family of products.

Further, it is an advantage of the present disclosure that such wireless control functionality can be connected to the main control circuitry (e.g., a printed circuit board assembly) if desired, or may be omitted if not; and such inclusion or omission may be done with little or no impact to other aspects of the system architecture.

**It** is a further advantage of the present disclosure that such wireless control functionality may be implemented without introducing electromagnetic or radio frequency (RF) interference to other aspects of the system. The present disclosure further allows to implement wireless control and wireless communication functionality to a communication device while maintaining electromagnetic shielding of the rest of the communication device. This has the advantage of avoiding interference from the components inside the communication device as well as vice versa introducing interference from the wireless interface in the communication device. Additionally, the present disclosure allows to implement wireless control and wireless communication functionality to a communication device while maintaining electromagnetic shielding of the rest of the communication device which preserves the communication device and the signals inside the communication from being compromised by external attacks.

Further, it is an advantage of the present disclosure that communication devices may be manufactured to provide wireless control functionality while maintaining efficient supply chains and manufacturing processes.

The present disclosure advantageously incorporates a wireless interface, such as a wireless chipset, into a modular button design to be used across different communication devices to allow modular assembly of the product depending on the customers requirements, thereby increasing design flexibility.

### BRIEF DESCRIPTION OF THE DRAWINGS

The above and other features and advantages of the present disclosure will become readily apparent to those skilled in the art by the following detailed description of example embodiments thereof, including those made with reference to the attached drawings, in which:
Fig. 1 is a block diagram illustrating an example communication device configured for modular wireless functionality control according to this disclosure,
Fig. 2 is a flow diagram of an example method of manufacturing a device configured for modular wireless functionality control according to this disclosure, and
Fig. 3 illustrates an example communication device configured for modular wireless functionality control according to this disclosure.

### DETAILED DESCRIPTION

Various example embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the disclosure or as a limitation on the scope of the disclosure. **In** addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described. Furthermore, definitions, ranges, materials, orientations, and further descriptions of various examples are provided.

A communication device is disclosed. The communication device may, for example, be a component or subsystem of a hearing protection apparatus, such as a headset, e.g., an over-the-ear type or an in-the-ear type hearing protection apparatus. In one or more examples, the communication device is a standalone unit rather than a subsystem of a hearing protection apparatus and may provide for wireless control functionality of another system. The hearing protection apparatus may comprise a hearing protection device. In one or more example communication devices, the communication may form part of a hearing protection system comprising the communication device, the hearing protection device, and a radio unit as disclosed herein. A hearing protection device may be, or may include, electronics and/or hardware configured to cancel, suppress, reduce, and/or mitigate environmental noise in order to protect the ear and/or associated internal organs that support a sense of hearing for the user of the hearing protection apparatus. The hearing protection apparatus may thus comprise mechanical noise suppression or cancellation, such as earmuffs or earplugs. In one or more examples, the hearing protection device comprises electronic noise suppression or cancellation, such as active noise cancellation technology, which may generate signals to destructively interfere with sound waves from the wearer's environment. **In** one or more examples, the hearing protection device comprises digital signal processing filters tuned or tunable to suppress, mitigate, or prohibit certain sound waves that come from the wearer's environment.

The communication device may include a processing unit or processing units. A processing unit or units may be or include general or special purpose semiconductor devices, which may control data or information exchanges within the communication device. A processing unit may be an application specific integrated circuit (ASIC), field programmable gate array (FPGA), computer processing unit (CPU) with one or more cores, a graphics processing unit (GPU), a general purpose GPU (GPGPU), or system on a chip (SoC). In one or more examples, a processing unit is a multi-IC package that includes one or more such devices.

The communication device may include a memory or memories of various types. The memory may be fixed and/or removable. Memory may be static or dynamic random access memory (SRAM or DRAM, respectively), NAND, and/or other volatile or non-volatile memory types. In one or more examples, a memory and processor unit are portions of a common semi-conductor device, such as (i) a die or multiple dies with memory and processing capability, (ii) a package on package (POP) assembly, and/or (iii) a multi-chip package (MCP). The processing unit may read from and/or write to memory.

The communication device may include a radio interface. The radio interface may be seen as an intermediary, which may be hardware or software, or a combination of both, that couples or provides a communication path between a processing unit or processing units and a radio unit or radio units. The radio interface may translate data or control signaling from one protocol to another. The radio interface may be or may include a codec. The radio interface may be a digital radio interface. In one or more examples, the radio interface comprises a radio connector, which may include terminals for wired connection to the radio unit or to multiple radio units. In other words, the radio connector may be configured to mechanically and/or electrically connect, such as releasably connect, the communication device to a radio unit.

The radio unit may be seen as one or more wireless chipsets and/or one or more RF chains, which may include processing resources and/or mechanical, electronic, software, and/or firmware to support wireless communication with the wireless communication device. In one or more examples, the radio interface may be referred to as a first interface or simply an interface.

The communication device may include a hearing protection device interface. The hearing protection device interface may be seen as an intermediary, which may be hardware or software, or a combination of both, that couples or provides a communication path between a processing unit and a hearing protection device. The hearing protection device interface may translate data or control signaling from one protocol to another. The hearing protection device interface may be or may include a codec, which may be the same as or different from another codec described herein. In one or more examples, the hearing protection device interface may be referred to as a second interface or simply an interface. The hearing protection device interface may comprise or be a hearing protection device connector. The hearing protection device connector may be configured to mechanically and/or electrically connect, such as releasably connect, the communication device to a hearing protection device.

The processing unit of the communication device may be configured to manage and/or control operations of or among the various components or subsystems of the communication device. By way of example, the processing unit may be configured to obtain (e.g., receive) a first input from the hearing protection device, e.g. via the hearing protection device interface, and/or transmit a first output to the hearing protection device, e.g. via the hearing protection device interface. Thus, this exchange and/or communication of first input and/or first output may be via the hearing protection device interface. The processing unit may be configured to obtain a second input from the radio unit, e.g. via the radio interface, and/or transmit a second output to the radio unit, e.g. via the radio interface.

**In** one or more examples, the communication device comprises a modular button assembly that may include a wireless interface. In other words, the wireless interface may be located inside the modular button. This for example allows to have a housing of the communication device to be electromagnetically shielded while the button assembly including the wireless interface is electromagnetically transparent. The modular button assembly may be seen as controlling functionality of communications between the processing unit and the radio unit via the radio interface. For example, the modular button assembly may allow for wireless communication between the processor unit and the radio unit and/or for wired communication between the processor unit and the radio unit.

Modular as used herein may refer to the manner of design and/or construction of the button assembly. In one or more examples, the communication device may be built and/or operated with or without the modular button assemble. In one or more examples, the communication device may be constructed or operated without a button assembly that controls wireless functionality. In one or more examples, the communication device may be constructed or operated with a button assembly that controls wireless functionality. A modular button assembly may thus be seen as allowing different examples or instances of the communication device to operate in similar fashion, with or without a button assembly. This may support effective manufacturing and supply chain operations in the production and/or distribution of communication devices according to various examples described herein.

The modular button module/assembly can incorporate a wireless functionality during product assembly if requested by the customer for that specific product. The wireless chipset and antenna placed in the button module and assembled and correctly sealed during production. Thus, the wireless functionality will not take up more space than the button module without the wireless functionality.

**In** one or more examples, a communication device for a hearing protection apparatus comprising a hearing protection device is disclosed, the communication device comprising a processing unit; a memory; a radio interface; and a hearing protection device interface, wherein the processing unit is configured to obtain a first input from the hearing protection device and/or transmit a first output to the hearing protection device via the hearing protection device interface, and wherein the radio interface comprises a radio connector, wherein the radio connector comprises terminals for wired connection to a radio unit, the processing unit is configured to obtain a second input from the radio unit and/or transmit a second output to the radio unit via the radio interface, wherein the communication device comprises a modular button assembly comprising a wireless interface.

The communication device may comprise a housing. The housing may be referred to, in one or more examples, as a/an package, enclosure, casing, shell, chassis, compartment, container, cabinet, cover, encasement, envelope, body, frame, structure, receptacle, case, assembly, shroud, encapsulation, mounting, or the like. The housing may be referred to, in one or more examples, as a means for housing or a housing means. The housing may comprise one or more recesses, such as a first recess and/or a second recess. The recess may be referred to, in one or more examples, as a/an opening, cavity, depression, indentation, hollow, concavity, void, groove, channel, alcove, aperture, notch, socket, well, pocket, cutout, depression, inset, niche, or the like. A recess, such as the first recess, may be a recess for arranging the modular button assembly. The (first) recess may be formed in the outer surface of the housing, e.g. in a first side surface of the housing.

In one or more examples, the communication device comprises a housing that may include a recess for arranging the modular button assembly.

The modular button assembly may be connected to the processing unit via the recess. Connected to, as used to describe the relationship between the modular button assembly and the processing unit, may be seen as a physical and/or electrical path that supports or allows for communication, power, and/or signaling exchange between the modular button assembly and the processing unit.

In one or more examples, the modular button assembly is connected to or configured to be connected to the processing unit via the recess, such as via a through-going opening in the recess.

In one or more examples, the modular button assembly comprises a cover. The cover may be electromagnetically transparent. The cover may be made of a rubber material.

Thus, the communication device, in one or more examples, may include a modular button assembly with a cover that is electromagnetically transparent. In other words, the cover may be permeable or penetrable by electromagnetic or RF signals. This may be seen as electromagnetically non-interfering or effectively non-reflective. Electromagnetic transparency may be understood in the context of the operation or use case of the communication device. In one or more examples, the cover may be electromagnetically transparent to wireless communication signals and/or to electromagnetic radiation in a frequency range of intended wireless communication of the modular button assembly. The cover may be referred to, in one or more examples, as a/an package, enclosure, casing, shell, chassis, compartment, container, cabinet, housing, encasement, envelope, body, frame, structure, receptacle, case, assembly, shroud, encapsulation, mounting, or the like. The cover may, in one or more examples, be referred to as a means for covering or a cover means.

In one or more examples, the housing of the communication device is electromagnetically sealed. Thus, the communication device may have a housing that is electromagnetically sealed. In other words, the housing may be impermeable or impenetrable by electromagnetic or RF signals. This may be seen as electromagnetically shielded, blocked, screened, or insulated. Electromagnetic sealing may be understood in the context of the operation or use case of the communication device. In one or more examples, the housing may be electromagnetically sealed to wireless communication signals and/or electromagnetic radiation in a frequency range of intended wireless communication of the modular button assembly.

The cover and housing described herein may each be constructed from one or more materials, which may be the same or different from one another, in order to provide electromagnetically transparent or electromagnetically sealed properties, performance, and/or behavior. Examples of such material that provide electromagnetic transparency include polymeric materials, ceramic materials, non-conductive composite material, and/or natural materials. Examples of such material that provide electromagnetic sealing include metallic material, conductive composites, and/or conductive coatings or films.

Non-limiting examples of polymeric materials providing electromagnetic transparency include polycarbonate, polyethylene, polypropylene, acrylonitrile butadiene styrene (ABS), polyamide, nylon, polyvinyl chloride (PVC), polymethyl methacrylate (PMMA or acrylic), and/or polyoxymethylene (POM or acetal). Non-limiting examples of ceramic materials providing electromagnetic transparency include alumina (aluminum oxide), silicon dioxide (quartz), boron nitride, glass-ceramics, ceramic composites, porcelain, silicon nitride, zirconia, cordierite, and/or beryllia. Non-limiting examples of composite materials providing electromagnetic transparency include glass-reinforced polymers, fiber-reinforced plastics, polymer composites, ceramic composites, organic composites, and/or thermoplastics. Non-exclusive examples of natural materials providing electromagnetic transparency include glass, crystals or crystalline structures, wood, paper, and/or rubber.

Non-limiting examples of metallic materials providing for electromagnetic sealing include copper, aluminum, steel, nickel, silver, gold, brass, zinc, tin, and/or various alloys that include one or more such materials. Non-limiting examples of conductive composite materials providing for electromagnetic sealing include carbon fiber, carbon polymer (or carbon-filled polymer), metalized fabrics, metal-filled plastic, graphite, graphene, and/or semiconductors. Non-limiting examples of conductive coatings or film providing for electromagnetic sealing include paints, depositions, tape, sputtering, plating, foil, or the like that include one or more conductive material.

To the extent the cover of the modular button assembly and the housing of the communication device are similar in certain aspects, they may be referred to, for example, as first and second. As a nonlimiting example, the housing may be referred to as a first package and the cover may be referred to as a second package. **In** such an example, the housing may be referred to as a first package (or other similar term) that is electromagnetically sealed and the cover may be referred to as a second package (or other similar term) this is electromagnetically transparent.

**In** one or more examples of the communication device, the modular button assembly is on a different side of the communication device than the radio interface and/or the hearing protection device interface. The relative location of the modular button assembly may be understood as a relative physical location with respect to other components. Additionally or alternatively, the relative locations of the modular button assembly on the one hand and the radio interface and/or the hearing protection device interface on the other hand, may be understood as different sides of the communication device having different electromagnetic transparency and/or sealing. The modular button assembly may be within a portion of the communication device that is electromagnetically transparent relative to one or more other components, while the radio interface and the hearing protection device interface may be in a portion of the communication device that is electromagnetically sealed relative to one or more other components. Or vice-versa.

Thus, the modular button assembly may be arranged on a first side of the housing, and the radio interface and/or the hearing protection device interface may be arranged on a second side of the housing. The first side of the housing may be perpendicular to or opposite the second side of the housing.

**In** one or more examples, the wireless interface of the modular button assembly comprises a wireless processing unit and an antenna. Thus, one or more examples of the communication device configured with a modular button assembly may include a wireless interface. The wireless interface may include a wireless processing unit and/or an antenna. The wireless processing unit may be in a common package with the antenna or portion of the antenna. Or the wireless processing unit may be connected to an antenna that is located elsewhere. The wireless processing unit may be or may include a wireless chipset. A wireless chipset may be seen as an integrated circuit assembly (such as a semiconductor or package of semiconductors) for wirele ss communication. It may be an SoC. It may include a modem, controller, and/or RF chain. It may be referred to as an RFIC. The wireless processing unit, for example, in combination with the antenna, may be capable of sending and/or receiving wireless data and/or control signals. It may be configured for signal processing. It may be configured for one or more specific protocols, such as IEEE 802.11 (Wifi), Bluetooth, IEEE 802.15.4 (Zigbee), Digital Enhanced Cordless Telecommunications (DECT), and/or a cellular protocol, such as one operating according to a 3GPP standard. One or more examples of a communication device may thus include a wireless interface configured to use a Bluetooth protocol (such as a Bluetooth Low Energy (BLE) protocol), or a DECT protocol, or both.

In one or more examples, the modular button assembly comprises a protrusion for receiving the antenna. The protrusion may be referred to as a/an projection, extension, prominence, outcropping, boss, tab, ridge, fin, rib, lug, extrusion, flange, or the like. It may be a feature that extends, projects, and/or protrudes out from another portion of the modular button assembly. It may be elevated and/or raised relative to other aspects of the modular button assembly. The protrusion may be configured for receiving the antenna because it may be or may include physical and/or electrical features or aspects for engagement, alignment, mounting, positioning, connecting, and/or securing the antenna. In one or more examples, the protrusion may be referred to as a means for receiving the antenna.

In one or more examples, a modular button assembly is mounted and/or sealed to the housing during production of the communication device. The terms mounted and mounting, as used in conjunction with the modular button assembly and/or in conjunction with methods of manufacturing such an assembly, may refer to a physical attachment method. In one or more examples, mounted may be understood as affixed, secured, attached, fastened, coupled, connected, joined, fixed, anchored, integrated, incorporated, installed, disposed, placed, located, adhered, and/or assembled. The terms sealed and sealing, as used in conjunction with mounting the modular button assembly and/or in conjunction with manufacturing such an assembly, may refer to causing the modular button assembly and/or the communication device to be impervious and/or resistant to ingress or egress of signals (such as RF signals), fluids, liquids, gases, particles, and/or contaminants. In one or more examples, mounting the modular button assembly means affixing the modular button assembly to the housing of the communication device, and sealing the modular button assembly means causing the modular button assembly and/or the housing to have a certain electromagnetic characteristic (e.g., electromagnetically transparent or electromagnetically sealed).

In one or more examples, the modular button assembly includes one or more buttons, each of which may be associated with one or more functions, e.g. of the communication device. Button, as used herein, may refer to a device operable by a user to activate, deactivate, modulate, change, and/or control a function, aspect, response, and/or operation of a communication device and/or a subsystem or component of the communication device and/or one or more units connected to the communication device. A button, for example, may be a physical input element, such as a/an actuator, input device, control element, manipulable element, interface, user interface, tactile device, manual control, and/or depressible member. A button may have, in one or more examples, a functional description or characteristic, such as user-actuatable, pressure-sensitive, pressure-actuated, touch-responsive, touch-sensitive, manually operated, force-receiving, and/or haptic. A button, for example, may have one or more operational characteristics, such as momentary contact, push-button, depression-activated, toggle/togglable, spring-biased, reciprocating, three-way, contact-making. A button may be, for example relative to other aspects of a cover or housing, raised, protruding, flush, recessed, and/or elevated, which may depend on a use case and/or operating state of a modular button assembly and/or of a communication device of which the modular button assembly is a component. A button may be a portion of a touch-screen user interface.

In one or more examples, the modular button assembly includes a first button, e.g. associated with a first function for activating and/or deactivating the wireless interface. In one or more examples, the modular button assembly includes a second button, e.g. associated with a second function, which may be independent from or different to the wireless interface. In one or more examples, the modular button assembly includes a switch button or a press button, or both.

The first button of the modular button module may be a hearing protection device control, e.g. via the wireless interface. In other words, the first button may be configured to control a functionality of the hearing protection device, such as program selection and/or volume, e.g. via the wireless interface. The second button of the modular button module may be a hearing protection device control different from the first button, e.g. via the wireless interface. In other words, the second button may be configured to control a functionality of the hearing protection device, such as program selection and/or volume, e.g. via the wireless interface.

The first button of the modular button module may be a radio unit control, e.g. via the wireless interface. In other words, the first button may be configured to control a functionality of the radio unit, such as push-to-talk, e.g. via the wireless interface. The second button of the modular button module may be a radio unit control different from the first button. In other words, the second button may be configured to control a functionality of the radio unit, such as canal selection and/or volume, e.g. via the wireless interface.

In one or more examples, the communication device comprises one or more secondary buttons, e.g. arranged on a second side and/or a third side of the housing. The first side of the housing may be perpendicular to or opposite the third side of the housing. The second side of the housing may be perpendicular to or opposite the third side of the housing. For example, a first secondary button and/or a second secondary button may be arranged on a third side of the housing. A secondary button may be associated with a function of the communication device and may be connected to the processing unit, e.g. for providing user input/control of the communication device and/or one or more units/devices connected to the communication device.

A method for manufacturing is also disclosed. For example, a communication device of a hearing protection apparatus, which may include a hearing protection device, a processing unit, a memory, a radio interface, and/or a hearing protection device interface may be manufactured according to such a method. In one or more examples, a communication device so manufactured may include a radio connector with terminals for wired connection to a radio unit. Additionally or alternatively, such a communication device may include a modular button assembly comprising a wireless interface. The method for manufacturing may include mounting and sealing the modular button assembly to a housing of the communication device. The skilled person will appreciate that the terms mounting and sealing, as well as other aspects of such a method and communication device, may be as described elsewhere herein.

The following figures are schematic and simplified for clarity, and they merely show details which aid understanding the disclosure, while other details have been left out.

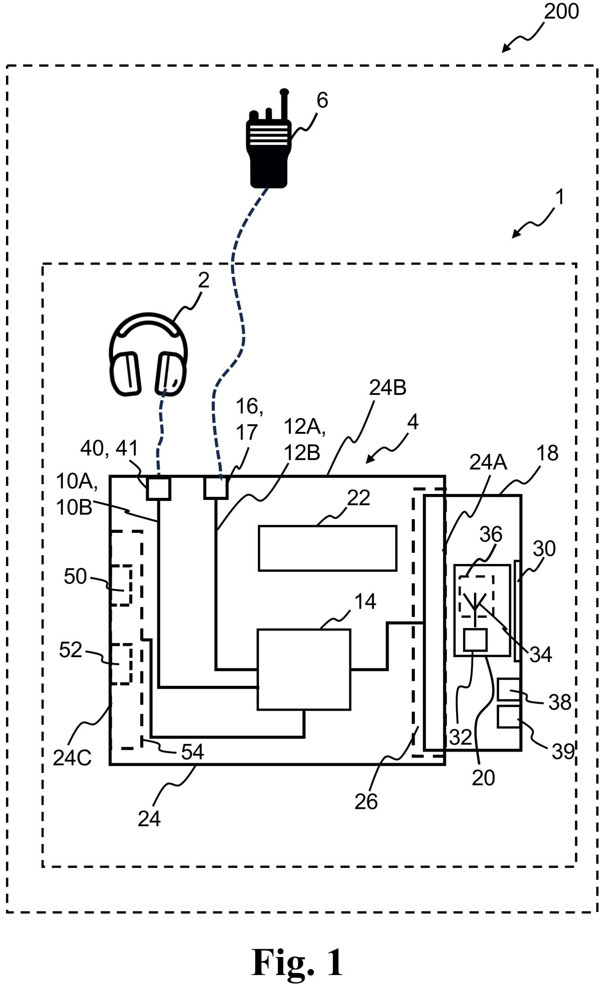

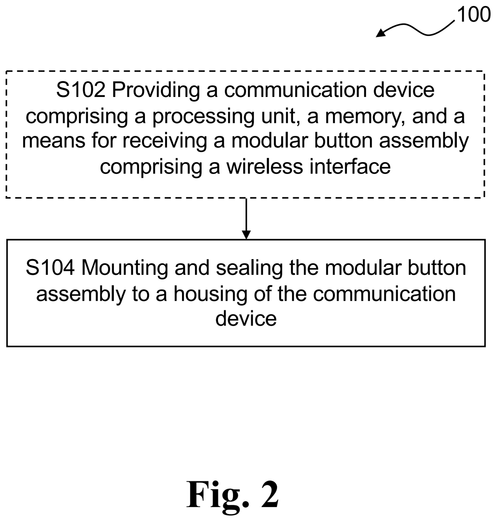

Throughout, the same reference numerals are used for identical or corresponding parts. The skilled person will recognize that the various definitions, functions, materials, components, and the like described above may be applied to and/or combined with the features described with reference to Fig. 1 and Fig. 2 and, for the sake of brevity, are not repeated in full.

Fig. 1 is a diagram illustrating an example communication device 4 configured for modular wireless functionality control according to this disclosure. For context, a hearing protection apparatus 1 is depicted, which may include the communication device 4 and/or a hearing protection device 2. A hearing protection system 200 is depicted, which may include the communication device 4, the hearing protection device 2, and a radio unit 6. The communication device 4 may include a processing unit 14, a memory 22, a radio interface 16, such as radio connector 17, and/or a hearing protection device interface 40, such as hearing protection device connector 41. In one or more examples, the communication device 4 comprises a modular button assembly 18. The modular button assembly 18 is arranged in a first recess 26 in a first side 24A of housing 24.

The processing unit 14 may be configured to obtain a first input 10A from the hearing protection device 2 and/or transmit a first output 10B to the hearing protection device 2 via the hearing protection device interface 40. The radio interface 16 may include a radio connector 17, which may comprise terminals for wired connection to a radio unit 6. The processing unit 14 may be configured to obtain a second input 12A from the radio unit and/or transmit a second output 12B to the radio unit via the radio interface 16. The hearing protection device interface 40 and/or the radio interface 16 are optionally arranged on a second side 24B of the housing 24.

The operations of the communication device 4 may be considered a method that the communication device 4 is configured to carry out. Also, while the described functions and operations may be implemented in software, such functionality may also (or alternatively) be carried out via dedicated hardware or firmware, or some combination of hardware, firmware and/or software.

Memory 22 may be one or more of a buffer, a flash memory, a hard drive, a removable media, a volatile memory, a non-volatile memory, a random access memory (RAM), or other suitable device, such as described elsewhere herein. **In** a typical arrangement, memory 22 may include a non-volatile memory for long term data storage and a volatile memory that functions as system memory for processing unit 14. Memory 22 may exchange data with processing unit 14 over a data bus. Control lines and an address bus between memory 22 and processing unit 14, as well as other components of communication device 4, also may be present (not shown in Fig. 1). Memory 22 is considered a non-transitory computer readable medium.

The communication device 4 includes a modular button assembly 18. **In** one or more examples, the modular button assembly 18 includes a wireless interface 20. The wireless interface may include a wireless processing unit 32 and/or an antenna 34. The wireless interface 20 may be configured to use one or more of: a Bluetooth protocol and a DECT protocol. Or the wireless interface 20 may be configured to use one or more of the various other protocols described herein. The modular button assembly 18, in one or more examples, comprises a protrusion 36. The protrusion may be for receiving the antenna 34.

In one or more examples, the communication device 4 comprises a housing 24. The housing 24 may include a recess 26, e.g. in a first side of the housing 24. The recess 26 may be used for arranging (e.g., coupling to, accepting, or connecting with) the modular button assembly 18. In one or more examples, the modular button assembly 18 is configured to be connected to (e.g., wired to, electrically connected with, and/or configured for electrical or electronic communication with) the processing unit 14 via the recess 26. In one or more examples, the housing 24 of the communication device 4 is electromagnetically sealed.

The modular button assembly 18 may include a cover 30. In one or more examples, the cover 30 is electromagnetically transparent. The modular button assembly 18 may be on one side, such as on first side 24A, or within one portion of the communication device 4, and the radio interface 16 and/or the hearing protection device interface 40 may be on another side, such as second side 24B, of the communication device 4. In one or more examples, the modular button assembly 18 is on a different side of the communication device 4 than the radio interface 16 and/or the hearing protection device interface 40. As described elsewhere herein, the modular button assembly 18 may be physically on a different side of the communication device 4 relative to the radio interface 16 and/or the hearing device interface 40, as schematically depicted in Fig. 1. Additionally or alternatively, the modular button assembly 18 may be located in a portion of the apparatus 1 that is electromagnetically transparent while other components, such as the radio interface 16 and/or the hearing device interface 40, may be located in a portion of the apparatus 1 that is electromagnetically sealed. Or vice versa.

In one or more examples of the communication device 4, the modular button assembly 18 is mounted and sealed to the housing 24 during production of the communication device 4. In one or more examples, the communication device 4 is produced and operated without the modular button assemble 18. The modular button assembly 18 may be separately provided (e.g., as an aftermarket accessory) and may be mounted and/or sealed following production of the communication device 4.

The modular button assembly 18 may optionally include one or more buttons, such as a first button 38 and/or a second button 39, which may be associated with one or more functions, respectively, of the communication device and/or one or more units/devices connected to the communication device, such as hearing protection device 2 and/or radio unit 6. In one or more examples, the modular button assembly 18 comprises a first button 38 associated with a first function. The first function may be for activating and/or deactivating the wireless interface 20, or a portion thereof, such as wireless processor 32 and/or antenna 34. In one or more examples. **In** one or more examples, the modular button assembly 18 comprises a second button 39 associated with a second function. The first function and/or the second function may be independent from the wireless interface 20. In one more examples, the first button 38 and/or the second button 39 controls other functionality of the communication device 4.

The communication device 4, such as within or as part of modular button assembly 18, may include a switch button or a press button, or both. In one or more examples, the communication device 4 includes more than one of each. **In** one or more examples, modular button assembly 18 comprises one or more of: a switch button and a press button. For example, the first button 38 may be a switch button and the second button 39 may be a press button, or vice versa. The first button 38 and the second button 39 may be the same type of button, including any of the various types of buttons mentioned herein.

The communication device 4 may comprise one or more secondary buttons including secondary first button 50 and/or secondary second button 52, e.g. forming part of user interface 54 of the communication device. Secondary first button 50 and/or secondary second button 52 are optionally configured to control functionality of the communication device, e.g. to activate push-to-talk functionality from the hearing protection device 2 to the radio unit 6. In other words, one or both secondary buttons 50, 52 may be associated with a function of the communication device and may be connected to the processing unit, e.g. for providing user input/control of the communication device and/or one or more units/devices connected to the communication device.

The secondary first button 50 and/or the secondary second button 52 may be arranged on a third side 24C of the housing, the third side 24C optionally opposite or perpendicular the first side 24A of the housing 24.

Fig. 2 shows a flow diagram of an example method of manufacturing a device according to the disclosure. The method 100 comprises, optionally, at S102, providing a communication device comprising a processing unit, a memory, and a means for receiving a modular button assembly comprising a wireless interface. A means for receiving a modular button assembly may be understood as a recess, such as described with reference to Fig. 1 or elsewhere herein.

The communication device may be a subsystem or component of a hearing protection apparatus, for example. In one or more examples, the communication device thus comprises a processing unit, a memory, a radio interface comprising a radio connector, and/or a hearing protection device interface. The radio connector may include terminals for wired connection to a radio unit. The hearing protection device interface may provide for connection to and/or communication with a hearing protection device.

The method 100 comprises, at S104, mounting and sealing the modular button assembly to a housing of the communication device. Mounting and sealing may be understood as defined elsewhere herein.

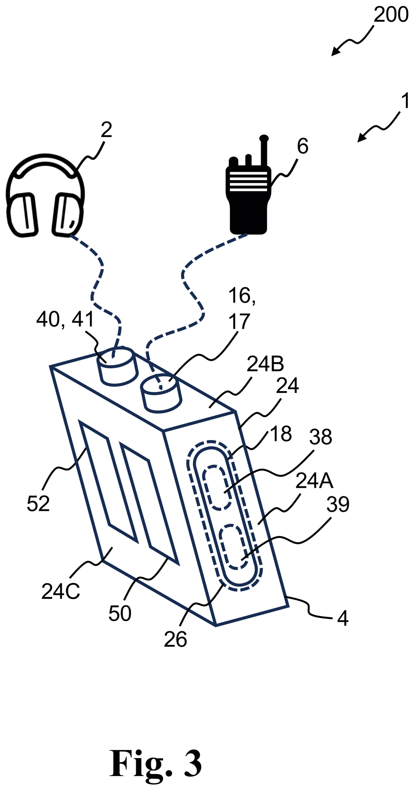

Fig. 3 is a perspective view of a example communication device 4 configured for modular wireless functionality control according to this disclosure. For context, a hearing protection apparatus 1 is depicted, which may include the communication device 4 and/or a hearing protection device 2. A hearing protection system 200 is depicted, which may include the communication device 4, the hearing protection device 2, and a radio unit 6. The hearing protection device 2 and radio unit 6 are wired to the communication device 4 via respective connectors 41, 17. The communication device 4 comprises modular button assembly 18 arranged in a first recess 26 in outer surface of a first side 24A of housing 24. The housing 24, e.g. the first side 24A, such as the first recess 26, of the communication device 4 is electromagnetically sealed. The radio connector 17 and the hearing protection device connector 41 are arranged on a second side 24B of the housing 24. The second side 24B may be perpendicular to (as illustrated) or opposite the first side 24A of the housing 24.

The communication device 4 may comprise secondary first button 50 and/or secondary second button 52 arranged on a third side 24C of the housing. The third side 24C may be perpendicular to (as illustrated) or opposite the first side 24A of the housing 24. The third side 24C may be perpendicular to (as illustrated) or opposite the second side 24B of the housing 24. The secondary first button 50 may be a volume control. The secondary second button 52 may be a push-to-talk control and/or a channel selection control. Thus, the secondary second button 52 may be a radio unit control. The secondary second button 52 may be a hearing protection device control. In other words, the secondary second button 52 may be configured to control a functionality of the hearing protection device 2.

Embodiments of products/devices and methods according to the disclosure are set out in the following items:
Item 1. A communication device for a hearing protection apparatus comprising a hearing protection device, the communication device comprising: a processing unit; a memory; a radio interface; and a hearing protection device interface, wherein the processing unit is configured to obtain a first input from the hearing protection device and/or transmit a first output to the hearing protection device via the hearing protection device interface, and wherein the radio interface comprises a radio connector, wherein the radio connector comprises terminals for wired connection to a radio unit, the processing unit is configured to obtain a second input from the radio unit and/or transmit a second output to the radio unit via the radio interface, wherein the communication device comprises a modular button assembly comprising a wireless interface.
Item 2. The communication device according to item 1, wherein the communication device comprises a housing comprising a recess for arranging the modular button assembly.
Item 3. The communication device according to item 2, wherein the modular button assembly is configured to be connected to the processing unit via the recess.
Item 4. The communication device according to any of the previous items, wherein the modular button assembly comprises a cover being electromagnetically transparent.
Item 5. The communication device according to any of items 2-4, wherein the housing of the communication device is electromagnetically sealed.
Item 6. The communication device according to any of the previous items, wherein the modular button assembly is on a different side of the communication device than the radio interface and the hearing protection device interface.
Item 7. The communication device according to any of the previous items, wherein the wireless interface comprises a wireless processing unit and/or an antenna.
Item 8. The communication device according to any of the previous items, wherein the modular button assembly comprises a protrusion for receiving the antenna.
Item 9. The communication device according to any of items 2-8, wherein the modular button assembly is mounted and/or sealed to the housing during production of the communication device.
Item 10. The communication device according to any of the previous items, wherein the modular button assembly comprises one or more buttons associated with one or more functions.
Item 11. The communication device according to item 10, wherein the modular button assembly comprises a first button associated with a first function for activating and/or deactivating the wireless interface.
Item 12. The communication device according to any of items 10-11, wherein the modular button assembly comprises a second button associated with a second function being independent from the wireless interface.
Item 13. The communication device according to any of the previous items, wherein the modular button assembly comprises one or more of: a switch button and a press button. Item 14. The communication device according to any of the previous items, wherein the wireless interface is configured to use one or more of: a Bluetooth protocol and a Digital Enhanced Cordless Telecommunications, DECT, protocol.
Item 15. A method for manufacturing a communication device of a hearing protection apparatus comprising a hearing protection device, the communication device comprising a processing unit, a memory, a radio interface comprising a radio connector, wherein the radio connector comprises terminals for wired connection to a radio unit, and a hearing protection device interface, wherein the communication device comprises a modular button assembly comprising a wireless interface, the method comprising: mounting and sealing the modular button assembly to a housing of the communication device.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

**It** may be appreciated that the figures comprise some components, circuitries, or operations which are illustrated with a solid line and some which are illustrated with a dashed line. Dashed lines are generally used to provide context and/or to depict further details or aspects not otherwise apparent from a 2D schematic. The depiction of solid or dashed lines, and the relationship to the various described embodiments, is to be understood in view of the corresponding description. Some connections between components are described but not depicted; the skilled person will recognize the interconnected or interoperability between components. **It** should be appreciated that these operations need not be performed in order presented. Furthermore, it should be appreciated that not all of the operations need to be performed. The example operations may be performed in any order and in any combination.

It is to be noted that the word "comprising" does not necessarily exclude the presence of other elements or steps than those listed.

It is to be noted that the words "a" or "an" preceding an element do not exclude the presence of a plurality of such elements.

It is to be noted that the term "indicative of" may be seen as "associated with", "related to", "descriptive of", "characterizing", and/or "defining". The terms "indicative of", "associated with", "related to", "descriptive of", "characterizing", and "defining" can be used interchangeably. The term "indicative of" can be seen as indicating a relation. For example, weight data indicative of weight may comprise one or more weight parameters.

It is to be noted that the word "based on" may be seen as "as a function of" and/or "derived from". The terms "based on" and "as a function of" can be used interchangeably. For example, a parameter determined "based on" a data set can be seen as a parameter determined "as a function of" the data set. In other words, the parameter may be an output of one or more functions with the data set as an input.

A list in the form of "A, B, or C, or any combination therefore" should be understood to mean "A", or "B", or "C", or "A and B", or "A and C", or "B and C", or "A and B and C".

A function may be characterizing a relation between an input and an output, such as mathematical relation, a database relation, a hardware relation, logical relation, and/or other suitable relations.

It should further be noted that any reference signs do not limit the scope of the claims, that the example embodiments may be implemented at least in part by means of both hardware and software, and that several "means", "units" or "devices" may be represented by the same item of hardware.

The various example methods, devices, nodes and systems described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program circuitries may include routines, programs, objects, components, data structures, etc. that perform specified tasks or implement specific abstract data types. Computer-executable instructions, associated data structures, and program circuitries represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly, to be regarded in an illustrative rather than restrictive sense. The claimed disclosure is intended to cover all alternatives, modifications, and equivalents.

### LIST OF REFERENCES

- 1: hearing protection apparatus
- 2: hearing protection device
- 4: communication device
- 6: radio unit
- 10A: first input from hearing protection device
- 10B: first output to hearing protection device
- 12A: second input from radio unit
- 12B: second output to radio unit
- 14: processing unit
- 16: radio interface
- 17: radio connector
- 18: modular button assembly
- 20: wireless interface
- 22: memory
- 24: housing
- 24A: first side of housing
- 24B: second side of housing
- 24C: third side of housing
- 26: recess
- 30: cover
- 32: wireless processing unit
- 34: antenna
- 36: protrusion
- 38: first button
- 39: second button
- 40: hearing protection device interface
- 41: hearing protection device connector
- 50: secondary first button
- 52: secondary second button
- 54: user interface
- 200: hearing protection system

## Claims

1. A communication device for a hearing protection apparatus comprising a hearing protection device, the communication device comprising:
a processing unit;
a memory;
a radio interface; and
a hearing protection device interface,
wherein the processing unit is configured to obtain a first input from the hearing protection device and/or transmit a first output to the hearing protection device via the hearing protection device interface, and wherein the radio interface comprises a radio connector, wherein the radio connector comprises terminals for wired connection to a radio unit, the processing unit is configured to obtain a second input from the radio unit and/or transmit a second output to the radio unit via the radio interface, wherein the communication device comprises a modular button assembly comprising a wireless interface.

2. The communication device according to claim 1, wherein the communication device comprises a housing comprising a recess for arranging the modular button assembly.

3. The communication device according to claim 2, wherein the modular button assembly is configured to be connected to the processing unit via the recess.

4. The communication device according to any of the previous claims, wherein the modular button assembly comprises a cover being electromagnetically transparent.

5. The communication device according to any of claims 2-4, wherein the housing of the communication device is electromagnetically sealed.

6. The communication device according to any of the previous claims, wherein the modular button assembly is on a different side of the communication device than the radio interface and the hearing protection device interface.

7. The communication device according to any of the previous claims, wherein the wireless interface comprises a wireless processing unit and an antenna.

8. The communication device according to any of the previous claims, wherein the modular button assembly comprises a protrusion for receiving the antenna.

9. The communication device according to any of claims 2-8, wherein the modular button assembly is mounted and sealed to the housing during production of the communication device.

10. The communication device according to any of the previous claims, wherein the modular button assembly comprises one or more buttons associated with one or more functions.

11. The communication device according to claim 10, wherein the modular button assembly comprises a first button associated with a first function for activating and deactivating the wireless interface.

12. The communication device according to any of claims 10-11, wherein the modular button assembly comprises a second button associated with a second function being independent from the wireless interface.

13. The communication device according to any of the previous claims, wherein the modular button assembly comprises one or more of: a switch button and a press button.

14. The communication device according to any of the previous claims, wherein the wireless interface is configured to use one or more of: a Bluetooth protocol and a Digital Enhanced Cordless Telecommunications, DECT, protocol.

15. A method for manufacturing a communication device of a hearing protection apparatus comprising a hearing protection device, the communication device comprising a processing unit, a memory, a radio interface comprising a radio connector, wherein the radio connector comprises terminals for wired connection to a radio unit, and a hearing protection device interface, wherein the communication device comprises a modular button assembly comprising a wireless interface, the method comprising:
- mounting and sealing (S104) the modular button assembly to a housing of the communication device.
